# FASCICULE DE BREVET EUROPEEN

(11) **EP 0 824 012 B1**
(45) Date de publication et mention de la délivrance du brevet: **19.11.2003**
(21) Numéro de dépôt: 97401882.2
(22) Date de dépôt: 05.08.1997
(51) Int. Cl.: A61F 2/06, A61B 17/11, F16L 31/00, F16L 31/02

(54) **Prothèse collerette**
Prothese mit Flansch
Prosthesis having collar portion

(30) Priorité: 05.08.1996 FR 9609874
(43) Date de publication de la demande: 18.02.1998
(73) Titulaire: Association René Leriche, 75014 Paris (FR)
(72) Inventeur: Zegdi, Rachid, 94230 Cachan (FR); Fabiani, Jean-Noel, 75014 Paris (FR)
(74) Mandataire: Remy, Vincent Noel Paul

(56) Documents cités:
- WO-A-87/04915
- US-A- 4 352 358
- US-A- 4 523 592
- US-A- 4 693 249
- US-A- 4 728 328
- US-A- 5 486 187

## Description

La présente invention concerne une prothèse vasculaire.

Les prothèses vasculaires qui sont utilisées en chirurgie vasculaire pour traiter des vaisseaux sanguins malades, notamment des portions d'artères, par pontage ou par substitution, sont des conduits sensiblement cylindriques qui peuvent, dans certains cas, comporter des ramifications.

Les prothèses couramment utilisées à ce jour sont obtenues par tissage ou tricotage de fibres polyester, par exemple celles connues sous l'appellation Dacron, le tissu ou le tricot étant ensuite revêtu d'une couche d'un matériau imperméable. On utilise également des prothèses vasculaires réalisées en polytétrafluoroéthylène (PTFE).

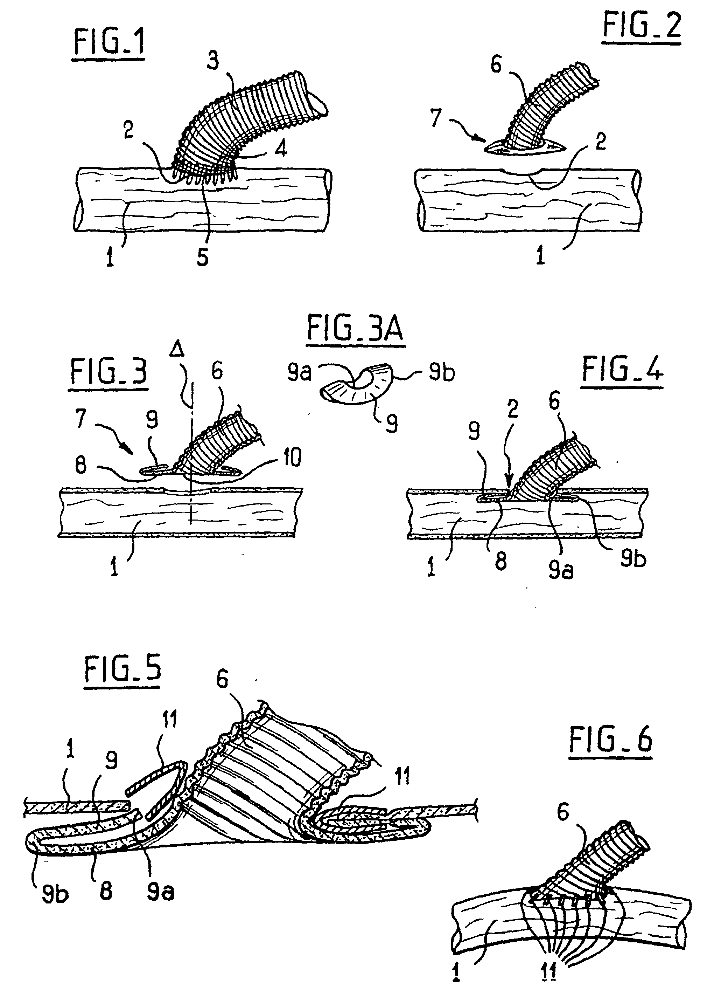

L'anastomose d'une prothèse vasculaire, c'est-à-dire sa mise en communication avec un vaisseau sanguin, s'effectue traditionnellement par abouchement de la prothèse et du vaisseau, c'est-à-dire par mise en regard de leurs embouchures respectives, puis par couture bord-à-bord desdites embouchures. La figure 1 donne une représentation schématique d'une telle anastomose.

Lorsque l'on procède au remplacement d'une portion malade d'un vaisseau, on exécute une anastomose termino-terminale, c'est-à-dire que la prothèse est placée dans le prolongement axial de la partie saine du vaisseau, à la place de la portion malade que l'on élimine.

Dans le cas d'un pontage, on procède à une anastomose terminolatérale, ce qui signifie que l'embouchure de la prothèse est raccordée latéralement au vaisseau, dans lequel on a préalablement pratiqué une lumière latérale à cet effet.

Dans les deux cas, la prothèse est cousue au vaisseau à l'aide d'un fil.

Cette technique chirurgicale, aujourd'hui assez bien maîtrisée, donne de bons résultats.

Toutefois, la réalisation des nombreux points de suture qui sont nécessaires pour effectuer l'anastomose d'une prothèse sur un vaisseau est une opération délicate à exécuter dans un espace de travail réduit, comme c'est le cas en particulier lorsqu'on utilise la technique d'endoscopie qui permet d'intervenir sur un vaisseau à travers une ouverture de taille réduite.

Dans ce cas, la durée de l'intervention chirurgicale est longuement prolongée, ce qui peut être difficile à supporter par certains patients.

Il peut également se produire que la couture d'une prothèse sur une artère malade soit très difficile à exécuter, par exemple lorsque la paroi de l'artère est calcifiée.

La présente invention vise à résoudre ces inconvénients en proposant une prothèse vasculaire qui peut être anastomosée sans couture sur un vaisseau, et ce, de manière particulièrement simple, rapide et fiable.

La présente invention a pour objet une prothèse vasculaire, telle que définie dans la revendication 1.

Au sens de la présente invention, on entend par nappe annulaire une nappe qui s'étend tout autour de l'extrémité de la prothèse, quelle que soit la forme de cette nappe.

La prothèse selon l'invention permet de s'affranchir de l'opération consistant à la coudre sur le vaisseau grâce à la présence de la nappe annulaire qui peut être solidarisée au vaisseau par clipsage ou par agrafage.

Des modes de réalisation particuliers sont définis dans les revendications dépendentes 2 à 4.

Une telle nappe se met en place sur un vaisseau sanguin comportant une lumière ou ouverture latérale en introduisant la partie de raccordement et la nappe dans ledit vaisseau à travers son ouverture et en tirant légèrement sur la prothèse pour appliquer la nappe contre la paroi intérieure du vaisseau autour de son ouverture, le bord libre de la nappe se situant au voisinage immédiat du pourtour de l'ouverture du vaisseau.

Ainsi, autour de son ouverture, la paroi vasculaire est en quelque sorte doublée intérieurement parla nappe.

On peut alors facilement solidariser la nappe à cette paroi vasculaire en pinçant celles-ci ensemble, par exemple à l'aide de clips qui sont de petits cavaliers que l'on engage à cheval sur la paroi vasculaire et sur la nappe, puis que l'on serre sur celles-ci en rapprochant les deux branches de chaque cavalier par déformation plastique.

Dans une autre variante, la nappe se situe à l'intérieur de la partie de raccordement. On solidarise ensuite la paroi vasculaire et la nappe en les pinçant ensemble. Ceci oblige à intervenir par l'intérieur de la prothèse, ce qui n'est pas exclu, compte tenu du fait que les moyens de solidarisation utilisés, à savoir par exemple les clips, peuvent être mis en place dans un espace de travail très réduit, et donc en particulier à l'intérieur même de la prothèse.

Naturellement, dans ce cas, il faut que la prothèse offre, au moment des opérations, un passage, différent de son embouchure, par lequel on peut introduire des clips et des instruments de travail.

Dans le but de mieux faire comprendre l'invention, on va en décrire maintenant différents modes de réalisation en référence au dessin annexé dans lequel :
- la figure 1 illustre l'état de la technique,
- la figure 2 représente l'extrémité d'une prothèse selon un premier mode de réalisation de l'invention lors de sa mise en place sur un vaisseau sanguin,
- la figure 3 est une vue en coupe de la figure 2,
- la figure 3A est une vue de détail de la figure 3,
- la figure 4 est une vue en coupe analogue à la figure 3, après positionnement de la prothèse,
- la figure 5 est une vue de détail de la figure 4, lors de la solidarisation de la prothèse au vaisseau,
- la figure 6 est une vue extérieure de la prothèse et du vaisseau anastomosés,
- la figure 7 est une vue en perspective d'une variante de prothèse selon le mode de réalisation de l'invention,
- la figure 8 est une vue en coupe représentant une autre variante du premier mode de réalisation de l'invention.

Sur la figure 1, on a représenté une artère 1 dans laquelle on a réalisé une lumière latérale 2.

Une prothèse vasculaire 3 est anastomosée à l'artère 1 par couture bord-à-bord de l'embouchure sensiblement elliptique 4 de la prothèse et de la lumière 2 de l'artère à l'aide d'un fil 5.

Cette technique d'anastomose correspond à l'état de la technique.

Sur la figure 2, on a représenté une artère 1, identique à la précédente, avec sa lumière latérale 2.

Une prothèse 6 selon un premier mode de réalisation est présentée en regard de la lumière 2 pour être anastomosée à l'artère 1.

La prothèse 6 comporte à son extrémité 7 des moyens pour son raccordement à l'artère 1 qui sont plus clairement visibles à la figure 3.

Lesdits moyens comprennent, d'une part, une partie du raccordement 8 prolongeant la paroi de la prothèse 6 de manière continue et une nappe annulaire 9 qui, dans le cas présent, est constituée par un prolongement replié sur lui-même de la partie de raccordement 8.

La partie de raccordement 8 et la nappe annulaire 9 s'étendent tout autour de l'embouchure 10 de la prothèse en étant conformées en une partie de cylindre.

La figure 3A montre la nappe annulaire 9 considérée isolément, qui présente bien une forme de cylindre et qui est délimitée intérieurement par un bord 9a et extérieurement par un bord 9b.

Au sens de l'invention, le bord 9a est le bord libre de la nappe. Si on le regarde dans l'axe **Δ** de l'embouchure 10 de la prothèse, le bord 9a présente une forme sensiblement elliptique.

Le bord 9b est le bord périphérique de la nappe.

Dans cet exemple, la partie de raccordement 8 et la nappe annulaire 9 sont réalisées dans le même matériau que le reste de la prothèse, à partir d'un tronçon d'extrémité thermoformé de cette dernière, mais il est clair qu'elles pourraient être réalisées séparément, puis réunies à la prothèse par collage ou soudage, la condition essentielle à respecter étant que le raccord entre la partie annulaire 9 et le reste de la prothèse soit continu, c'est-à-dire qu'il ne comporte aucun orifice.

Comme on le voit à la figure 4, on met en place la prothèse en introduisant la partie de raccordement 8 et la nappe annulaire 9 dans la lumière 2, puis en tirant légèrement sur la prothèse pour appliquer ladite nappe annulaire 9 contre la paroi intérieure de l'artère, autour de la lumière 2.

Le bord libre 9a se situe alors au voisinage du pourtour de ladite lumière.

Il reste ensuite à solidariser la prothèse à l'artère, ce qui peut être effectué à l'aide de clips 11 du type de ceux représentés à la figure 5.

Les clips peuvent être facilement insérés à cheval sur la paroi du vaisseau 1 et sur la nappe annulaire 9, comme on le voit à gauche sur la figure 5, puis serrés pour les pincer ensemble, comme représenté à droite sur la figure 5.

On dispose des clips 11 de cette manière tout autour de la lumière 2, en quantité suffisante pour assurer une bonne solidarisation de la prothèse sur l'artère.

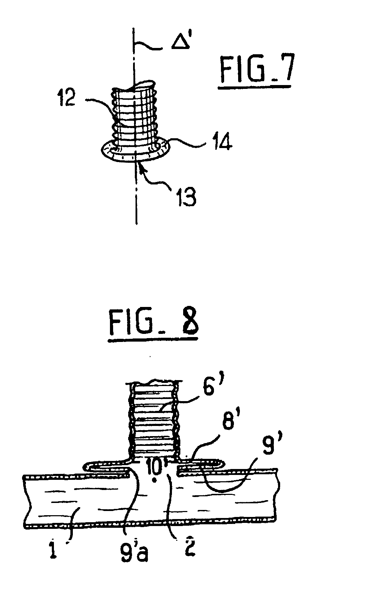

A la figure 7, on a représenté une prothèse 12 qui est destinée, comme celle du mode de réalisation précédent, à être raccordée latéralement à un vaisseau sanguin, mais qui, à la différence de la précédente, se raccorde à ce vaisseau, non pas obliquement, mais perpendiculairement.

Pour cette raison, l'embouchure 13 de la prothèse n'est pas elliptique mais sensiblement circulaire.

La prothèse comporte alors une nappe annulaire 14 qui, vue dans l'axe Δ' de l'ouverture 13, présente une forme circulaire.

En réalité, comme précédemment, la nappe annulaire 14 est une partie de cylindre du type de celle représentée à la figure 3A, ce qui signifie qu'elle est conformée en un anneau prenant appui sur la paroi latérale d'un cylindre.

La figure 8 représente une variante du mode de réalisation des figures 2 à 6.

Dans cette variante, la nappe annulaire 9' de la prothèse 6' se situe, non pas à l'extérieur de la partie de raccordement 8', mais à l'intérieur de cette dernière.

La mise en place de la prothèse sur l'artère s'effectue alors par simple appui contre la paroi extérieure du vaisseau, après mise en regard de l'embouchure 10' de la prothèse avec la lumière latérale 2 de l'artère.

On vient ensuite solidariser la paroi de l'artère avec la nappe annulaire 9' en mettant en place des clips (non représentés) similaires aux clips 11 précédemment décrits, mais cette fois par l'intérieur de la prothèse.

Ces clips peuvent être positionnés et serrés par exemple par endoscopie à l'intérieur de la prothèse.

La variante de la figure 8 peut être indiquée lorsque l'on souhaite traiter des vaisseaux sanguins dont le diamètre est insuffisant pour supporter intérieurement la présence de la nappe annulaire 9 ou 16.

Sur la figure 8, on a utilisé les mêmes références que précédemment, additionnées du signe ' , pour désigner les mêmes éléments.

Il est bien entendu que les modes de réalisation qui viennent d'être décrits ne présentent aucun caractère limitatif et qu'ils pourront recevoir toutes modifications désirables sans sortir pour cela du cadre de l'invention.

En particulier, il faut noter que, bien que l'invention ait été décrite en rapport avec une prothèse anastomosée sur un vaisseau sanguin naturel, elle concerne également une prothèse qui peut être anastomosée sur une autre prothèse, laquelle constitue aussi un conduit au sens de la présente invention, au même titre qu'un vaisseau sanguin naturel.

## Revendications

1. Prothèse vasculaire, destinée à réaliser une anastomose latéro-terminale sur un conduit tubulaire (1) muni d'une ouverture latérale (2) comportant, à l'une au moins des ses extrémités, une nappe annulaire (9, 9') délimitée par un bord libre (9a, 9'a) destiné à être positionné au voisinage immédiat de l'ouverture (2) du conduit, **caractérisée par le fait que** ladite nappe (9, 9') est conformée de manière à pouvoir s'appliquer autour de l'ouverture (2) du conduit contre la paroi intérieure ou extérieure de ce dernier, tout en conservant son bord libre (9a, 9'a) au voisinage immédiat du pourtour de l'ouverture (2) du conduit, et **par le fait que** ladite nappe annulaire (9, 9') est en forme de partie latérale de cylindre, délimitée extérieurement par un bord périphérique (9b) correspondant sensiblement à l'intersection de ce cylindre avec un autre cylindre, et délimitée intérieurement par son bord libre (9a, 9'a) qui définit un orifice correspondant à l'embouchure (10, 10') de la prothèse.

2. Prothèse selon la revendication 1, **caractérisée par le fait que** la nappe annulaire (9, 9') est raccordée à l'extrémité de la prothèse par une partie de raccordement (8, 8') qui prend naissance à l'extrémité de la prothèse et s'étend jusqu'au bord périphérique (9b) de la nappe, ladite partie de raccordement présentant sensiblement la même forme que la nappe de sorte que le bord libre (9a, 9'a) de la nappe se situe au voisinage de la naissance de la partie de raccordement, à l'extrémité de la prothèse.

3. Prothèse selon la revendication 2, **caractérisée par le fait que** la nappe (9) se situe à l'extérieur de la partie de raccordement (8), de sorte que l'extrémité de la prothèse traverse l'orifice délimité par le bord libre (9a) de la nappe et se prolonge radialement par la partie de raccordement (8) jusqu'au bord périphérique (9b) de la nappe, laquelle recouvre la partie de raccordement.

4. Prothèse selon la revendication 2, **caractérisée par le fait que** la nappe (9') se situe à l'intérieur de la partie de raccordement (8').

## Patentansprüche

1. Prothese für ein Gerät zum Realisieren einer lateral anschließenden Anastomose (Gefäßverbindung) an eine rohrförmige Leitung (1), die mit einer lateralen Öffnung (2) versehen ist, umfassend an wenigstens einem der Enden eine ringförmige Anlage (9, 9'), die durch einen freien Rand (9a, 9'a) begrenzt ist, der in unmittelbarer Nachbarschaft zur Öffnung (2) der Leitung positionierbar ist, **dadurch gekennzeichnet, daß** die Anlage (9, 9') derart ausgebildet ist, daß sie sich um die Öffnung (2) der Leitung gegen die Innenwand oder Außenwand letzterer anlegen kann, wobei der freie Rand (9a, 9'a) der Anlage in unmittelbarer Nachbarschaft des äußeren Umfangs der Öffnung (2) der Leitung verbleibt, und daß die ringförmige Anlage (9, 9') in Form eines lateralen Stücks eines Zylinders ausgebildet ist und außen durch einen Umfangsrand (9b), der im wesentlichen dem Übergang dieses Zylinders zu einem anderen Zylinder entspricht, und innen durch ihren freien Rand (9a, 9'a) begrenzt ist, der ein Loch definiert, das der Mündung der Prothese (10, 10') entspricht.

2. Prothese nach Anspruch 1, **dadurch gekennzeichnet, daß** die ringförmige Anlage (9, 9') mit dem Ende der Prothese durch ein Verbindungsteil (8, 8') verbunden ist, das an dem Ende der Prothese seinen Anfang nimmt und sich bis zum Außenumfang (9b) der Anlage erstreckt, wobei das Verbindungsstück im wesentlichen die gleiche Form wie die Anlage aufweist, so daß der freie Rand (9a, 9'a) der Anlage benachbart dem Anfang des Verbindungsstücks am Ende der Prothese liegt.

3. Prothese nach Anspruch 2, **dadurch gekennzeichnet, daß** die Anlage (9) an der Außenseite des Verbindungsstücks (8) liegt, so daß das Ende der Prothese das von dem freien Rand (9a) der Anlage begrenzte Loch durchdringt und durch das Verbindungsstück (8) bis zum Umfangsrand (9b) der Anlage radial verlängert ist, die das Verbindungsstück bedeckt.

4. Prothese nach Anspruch 2, **dadurch gekennzeichnet, daß** die Anlage (9') im Inneren des Verbindungsstücks (8') liegt.

## Claims

1. A vascular prosthesis for implementing a side-to-end anastomosis on a tubular duct (1) provided with a side opening (2), the prosthesis having at at least one of its ends an annular sheet (9, 9') defined by a free edge (9a, 9'a) for being positioned in the immediate vicinity of the opening (2) of the duct, the prosthesis being **characterized by** the fact that said sheet (9, 9') is shaped in such a manner as to be capable of pressing around the opening (2) in the duct against the inside or outside wall thereof while keeping its free edge (9a, 9'a) in the immediate vicinity of the periphery of the opening (2) of the duct, and by the fact that said annular sheet (9a, 9'a) is in the form of a side portion of a cylinder, being defined externally by a peripheral edge (9b) corresponding substantially to the intersection of said cylinder with another cylinder, and defined internally by its free edge (9a, 9'a) which defines an opening corresponding to the mouth (10, 10') of the prosthesis.

2. A prosthesis according to claim 1, **characterized by** the fact that the annular sheet (9, 9') is connected to the end of the prosthesis by a connection portion (8, 8') which starts from the end of the prosthesis and extends as far as the peripheral edge (9b) of the sheet, said connection portion presenting substantially the same shape as the sheet so that the free edge (9a, 9'a) of the sheet is situated in the vicinity of the start of the connection portion at the end of the prosthesis.

3. A prosthesis according to claim 2, **characterized by** the fact that the sheet (9) is situated outside the connection portion (8) so that the end of the prosthesis passes through the orifice defined by the free edge (9a) of the sheet and extends radially by means of the connection portion (8) as far as the peripheral edge (9b) of the sheet, which sheet covers the connection portion.

4. A prosthesis according to claim 2, **characterized by** the fact that the sheet (9') is situated inside the connection portion (8').
